# EUROPEAN PATENT SPECIFICATION

(11) **EP 3 825 099 B1**
(45) Date of publication and mention of the grant of the patent: **22.10.2025**
(21) Application number: 19838418.2
(22) Date of filing: 19.04.2019
(51) Int. Cl.: C08F 283/00, C08L 51/08, B29C 64/106, A61C 7/08, B33Y 10/00, B33Y 70/00

(54) **THREE-DIMENSIONAL PRINTING COMPOSITION AND METHOD OF PRODUCING DENTAL ARTICLE**
ZUSAMMENSETZUNG ZUR DREIDIMENSIONALEN DRUCK UND VERFAHREN ZUR HERSTELLUNG VON DENTALARTIKELN
COMPOSITION D'IMPRESSION TRIDIMENSIONNELLE ET PROCÉDÉ DE PRODUCTION D'ARTICLE DENTAIRE

(30) Priority: 19.07.2018 JP 2018136108
(43) Date of publication of application: 26.05.2021
(73) Proprietor: GC Corporation, Sunto-gun, Shizuoka 410-1307 (JP)
(72) Inventor: TAKADA, Daisuke, Tokyo 174-8585 (JP); KARIYA, Syuji, Tokyo 174-8585 (JP)
(74) Representative: Müller-Boré & Partner Patentanwälte PartG mbB
(86) International application number: PCT/JP2019/016834
(87) International publication number: WO 2020/017122

(56) References cited:
- WO-A1-2014/081004
- WO-A1-2016/098636
- WO-A1-2018/038056
- WO-A1-2018/105463
- JP-A- 2009 178 348
- JP-A- 2016 179 638
- US-A- 6 136 881
- US-A1- 2018 110 683

## Description

### FIELD OF THE INVENTION

The present invention relates to a three-dimensional printing composition and a method of producing a dental article.

### BACKGROUND OF THE INVENTION

In recent years, developments have been made in three-dimensional printing technologies. In the field of dentistry, denture bases, artificial teeth, and other dental articles have also been produced by three-dimensional printings (see, for example, Patent Document 1) .

In contrast, clear resin aligners (mouthpieces) are used for orthodontics.

### RELATED-ART DOCUMENT

### Patent Documents

Patent Document 1: International Publication No. 2014/172716

US 6 136 881 discloses light-hardenable orthodontic resin compositions comprising a urethane bond-free (meth)acrylate, a urethane bond-containing (meth)acrylate and polyurethane powder.

US 2018/110683 discloses resin compositions comprising urethane bond-containing (meth)acrylate and polyurethane powder.

### SUMMARY OF THE INVENTION

### Problems to be Solved by the Invention

However, there is a problem that when an orthodontic aligner is produced by three-dimensional printing, cracks are likely to generate in the orthodontic aligner during attachment and detachment.

One aspect is to provide a three-dimensional printing composition capable of suppressing cracking during attachment and detachment even when an orthodontic aligner is produced by three-dimensional printing.

### Means for Solving the Problems

One aspect is to provide a three-dimensional printing composition containing: 10 to 80% by mass of (meth)acrylate having a urethane bond; 10 to 80% by mass of (meth)acrylate free from a urethane bond; 3 to 30% by mass of a polyurethane powder; and 0.01 to 10% by mass of a photopolymerization initiator, wherein the viscosity of the three-dimensional printing composition measured using a type B viscometer at 23°C is 200 to 3,000 mPa·s.

### Effects of the Invention

According to an aspect of the invention, a three-dimensional printing composition capable of suppressing cracking during attachment and detachment even when an orthodontic aligner is produced by three-dimensional printing.

### DETAILED DESCRIPTION OF THE INVENTION

Next, an embodiment for carrying out the present invention will be described.

### [Three-Dimensional Printing Composition]

A three-dimensional printing composition of the present embodiment contains (meth)acrylate having a urethane bond, (meth)acrylate free from a urethane bond, a polyurethane powder, and a photopolymerization initiator.

The (meth)acrylate refers to a compound (e.g., a monomer, oligomer, prepolymer, or the like) having a methacryloyloxy group and/or an acryloyloxy group (hereinafter referred to as (meth)acryloyloxy group).

The (meth)acrylate having a urethane bond preferably has two or more (meth)acryloyloxy groups, and particularly preferably has two (meth)acryloyloxy groups.

Examples of (meth)acrylates having a urethane bond include bis(2-(meth)acryloyloxyethyl)-2,2,4-trimethylhexamethylene dicarbamate; 1,3,5-tris[1,3-bis{(meth)acryloyloxyl-2-propoxycarbonylaminohexane]-1,3,5-(1H,3H,5H)triazine-2,4,6-trion; (meth)acrylate of urethane oligomers formed from 2,2'-bis(4-hydroxycyclohexyl)propane, 2-oxypanone, hexamethylene diisocyanate, and 2-hydroxyethylene(meth)acrylate; (meth)acrylate of urethane oligomers formed from 1,3-butanediol, hexamethylene diisocyanate, and 2-hydroxyethyl (meth)acrylate; and the like. Two or more kinds of (meth)acrylate having urethan bond may be used in combination.

A content of the (meth)acrylate having a urethane bond in a three-dimensional printing composition of the present embodiment is 10 to 80% by mass and preferably 25 to 75% by mass. When the content of (meth)acrylate having a urethane bond in the three-dimensional printing composition is less than 10% by mass, a polyurethane powder is easily precipitated, and a storage stability of the three-dimensional printing composition decreases. When the content of (meth)acrylate having a urethane bond in the three-dimensional printing composition is more than 80% by mass, a viscosity of the three-dimensional printing composition increases, and thus a dental article is not easily producible by three-dimensional printing.

A (meth)acrylate free from a urethane bond preferably has two or more (meth)acryloyloxy groups, and particularly preferably has two (meth)acryloyloxy groups.

Examples of (meth)acrylates free from a urethane bond include methyl (meth)acrylate, ethyl (meth)acrylate, isopropyl (meth)acrylate, n-butyl (meth)acrylate, isobutyl (meth)acrylate, hydroxypropyl (meth)acrylate, tetrahydrofurfuryl (meth)acrylate, glycidyl (meth)acrylate, 2-hydroxyethyl (meth)acrylate, 2-hydroxypropyl (meth)acrylate, 3-hydroxypropyl (meth)acrylate, 2-ethoxyethyl (meth)acrylate, 2-methoxyethyl (meth)acrylate, 2-ethylhexyl (meth)acrylate, benzyl (meth)acrylate, 2-hydroxy-1,3-di(meth)acryloxypropane, ethylene glycol di(meth)acrylate, diethylene glycol di(meth)acrylate, triethylene glycol di(meth)acrylate, butylene glycol di(meth) acrylate, neopentyl glycol di(meth) acrylate, 1,3-butanediol di(meth)acrylate, 1,4-butanediol di(meth)acrylate, 1,6-hexanediol di(meth)acrylate, trimethylol propane tri(meth)acrylate, trimethylol ethane tri(meth)acrylate, pentaerythritol tri(meth)acrylate, trimethylol methane tri(meth)acrylate, pentaerythritol tetra(meth)acrylate, polybutylene glycol di(meth)acrylate, ethoxylated bisphenol A di(meth)acrylate, bisphenol A diglycidyl (meth)acrylate, and the like. Two or more kinds may be used in combination.

A content of the (meth)acrylate free from a urethane bond in the three-dimensional printing composition of the present embodiment is 10 to 80% by mass and preferably 25 to 75% by mass. When the content of (meth)acrylate free from a urethane bond in the three-dimensional printing composition is less than 10% by mass, a viscosity of the three-dimensional printing composition increases, and thus a dental article is easily producible by three-dimensional printing. When the content of (meth)acrylate free from a urethane bond in the three-dimensional printing composition is more than 80% by mass, a polyurethane powder is easily to be precipitated, and a storage stability of the three-dimensional printing composition decreases.

A volume average particle size of the polyurethane powder is preferably 1 to 20 µm and more preferably 3 to 15 µm. When the volume average particle size of the polyurethane powder is 1 µm or more, the viscosity of the three-dimensional printing composition of the present embodiment is further reduced, and thus a dental article is easily producible by three-dimensional printing. When the volume average particle size is 20 µm or less, the polyurethane powder is not easily precipitated, and a storage stability of the three-dimensional printing composition of the present embodiment further increases.

A content of the polyurethane powder in the three-dimensional printing composition of the present embodiment is 3 to 30% by mass, preferably 4 to 25% by mass, and more preferably 5 to 20% by mass. When the content of the polyurethane powder in the three-dimensional printing composition is less than 3% by mass, cracks are likely to generate in the orthodontic aligner during attachment and detachment even when an orthodontic aligner is produced by three-dimensional printing. When the content of the polyurethane powder in the three-dimensional printing composition is more than 30% by mass, a dental article is not easily producible by three-dimensional printing, because the viscosity of the three-dimensional printing composition increases.

Examples of photopolymerization initiators include camphorquinone, benzyl, diacetyl, benzyl dimethyl ketal, benzyl diethyl ketal, benzyl di(2-methoxyethyl)ketal, 4,4'-dimethyl (benzyl dimethyl ketal), anthraquinone, 1-chloroanthraquinone, 2-chloroanthraquinone, 1,2-benzanthraquinone, 1-hydroxyanthraquinone, 1-methylanthraquinone, 2-ethylanthraquinone, 1-bromoanthraquinone, thioxanthone, 2-isopropylthioxanthone, 2-nitrothioxanthone, 2-methylthioxanthone, 2,4-dimethylthioxantone, 2,4-diethylthioxanthone, 2,4-diisopropylthioxanthonton, 2-chloro-7-trifluoromethylthioxanthone, thioxanthone-10, 10-dioxide, thioxanthone-10-oxide, benzoin methyl ether, benzoin ethyl ether, isopropyl ether, benzoin isobutyl ether, benzophenone, bis(4-dimethylaminophenyl)ketone, 4,4'-bis(diethylamino)benzophenone, (2,4,6-trimethylbenzoyl)diphenylphosphine oxide (TPO), bis(2,4,6-trimethylbenzoyl)phenylphosphine oxide, and the like. Two or more kinds may be used in combination.

A content of the photopolymerization initiator in the three-dimensional printing composition of the present embodiment is preferably 0.01 to 10% by mass and more preferably 0.1 to 5% by mass. When the content of the photopolymerization initiator in the three-dimensional printing composition is less than 0.01% by mass, and thus, the three-dimensional printing composition is not easily photo-cured. When the content of the photopolymerization initiator in the three-dimensional printing composition is more than 10% by mass, a dental article is more likely to become discolored even when the dental article is produced by three-dimensional printing.

The three-dimensional printing composition of the present embodiment may further contain fillers, pigments, UV absorbers, polymerization inhibitors, stabilizers, viscosity modifiers, and the like.

Forms of the three-dimensional printing composition of the present embodiment include liquids, pastes, and the like.

The viscosity of the three-dimensional printing composition measured using a type B viscometer at 23°C is 200 to 3,000 mPa·s and preferably 300 to 2,000 mPa·s. When the viscosity of the three-dimensional printing composition is less than 200 mPa·s or the viscosity thereof is more than 3,000 mPa·s, and thus, a dental article is not easily producible by three-dimensional printing.

Examples of dental articles include dental prostheses, orthodontic aligners, orthodontic retainers, surgical guides, splints (mouthpieces), indirect bonding trays, dental models. Of these, orthodontic aligners are preferred.

### [Method of Producing Dental Articles]

A method of producing a dental article according to the present embodiment includes a step of producing the dental article by three-dimensional printing using the three-dimensional printing composition of the present embodiment.

A publicly-known three-dimensional printer can be used in three-dimensional printing of dental articles using the three-dimensional printing composition of the present embodiment.

Examples of three-dimensional printing methods include a stereolithography (SLA) method, a digital light processing (DLP) method, and the like. Among these, the DLP method is preferably used.

A commercially available product of a DLP-type three-dimensional printer includes MAX385 (manufactured by ASIGA) and the like.

A method of three-dimensionally printing a dental article using the three-dimensional printing composition of the present embodiment includes, for example, a method of irradiating light from above a container containing a three-dimensional printing composition (a free liquid surface method) or a method of irradiating light from below a container (a regulated liquid level method). Among these, the regulated liquid level method is preferably applied.

When a dental article is produced by three-dimensional printing using the regulated liquid level method, the bottom surface of the container is light transmissive and light radiated from below the container passes through the bottom surface of the container and causing the three-dimensional printing composition to be irradiated with light.

Examples of light that the three-dimensional printing composition is irradiated with when a dental article is three-dimensionally produced by using a three-dimensional printing article of the present embodiment include, for example, ultraviolet light, visible light, or the like having a wavelength of 380 to 450 nm.

Examples of light sources of light radiated into the three-dimensional printing composition include LED lasers, LED lamps, LED projectors, and the like.

The method of producing a dental article of the present embodiment may further include a step of cleaning a three-dimensional printing article, a step of post-polymerizing a three-dimensional printing article, and the like.

The dental article produced by three-dimensional printing using the three-dimensional printing composition of the present embodiment preferably has a three-point bending strength of 10 to 80 MPa, an elastic modulus of 0.2 to 1.8 GPa, and a strain of 12 to 20%.

### EXAMPLE

Hereinafter, examples of the present invention will be described, but the present invention is not limited to the examples.

### [Examples 1 to 6, Comparative Examples 1 to 4]

A three-dimensional printing paste was prepared by mixing (meth)acrylate having a urethane bond with (meth)acrylate free from a urethane bond, a polyurethane powder, a photopolymerization initiator, and a polymerization inhibitor in a compounding amount (% by mass) indicated in Table 1.

The abbreviations in Table 1 are as follows.
UDMA: Bis(2-methacryloyloxyethyl)-2,2,4-trimethylhexamethylene dicarbamate ((meth)acrylate having a urethane bond)
Biss MEPP 1: Ethoxylated bisphenol A dimethacrylate BPE-100 (manufactured by Shin-Nakamura Chemical Co., Ltd.) ((meth)acrylate free from a urethane bond)
Biss MEPP 2: Ethoxylated bisphenol A dimethacrylate BPE-500 (manufactured by Shin-Nakamura Chemical Co., Ltd.) ((meth)acrylate free from a urethane bond)
TEGDMA: Triethylene glycol dimethacrylate ((meth)acrylate free from a urethane bond)
TPO (2,4,6-trimethylbenzoyl) diphenylphosphine oxide (photopolymerization initiator)
BHT: Dibutyl hydroxytoluene (polymerization inhibitor)
Polyurethane powder (C-400): Urethane beads C-400 having a volume average particle diameter of 15 µm (manufactured by Negami Chemical Industrial Co., Ltd.)
Polyurethane powder (C-1000): Urethane beads C-1000 having a volume average particle diameter of 3 µm (manufactured by Negami Chemical Industrial Co., Ltd.) (Viscosity)

The viscosity of three-dimensional printing paste was measured using type B viscometer at 23°C.

### [Preparation of Orthodontic Aligners]

After a shape of an orthodontic aligner conforming to a tooth model was designed using the CAD Software Composer (manufactured by ASIGA), an orthodontic aligner was three-dimensionally produced by the DLP-typed three-dimensional printer MAX385 (manufactured by ASIGA) and the three-dimensional printing paste. Next, the three-dimensional printing article was thoroughly washed with isopropanol, and then post-polymerized using a dental photopolymerize device to prepare the orthodontic aligner.

### [Testing for Cracks in Orthodontic Aligners]

The orthodontic aligner was attached and detached from the tooth model and visually checked for cracks. The crack test was judged based on the following criteria.
Excellent: No cracks were observed.
Poor: Cracks were observed.

### (Three-Point Bending Test of Test Piece)

After a test piece of 2 mm × 2 mm × 25 mm was designed using the CAD Software Composer (manufactured by ASIGA), the test piece was three-dimensionally produced using the DLP-typed three-dimensional printer MAX385 (manufactured by ASIGA) and the three-dimensional printing paste. Next, the three-dimensional printing article was thoroughly washed with isopropanol, and then post-polymerized using a dental photopolymerizer to prepare the test piece.

The test piece was polished using water-resistant polishing paper and stored at 37°C in water for 24 hours. Next, a three-point bending test of the test piece was performed at a crosshead speed of 1 mm/min using a small desktop tester EZ test (manufactured by Shimadzu Corporation), and the three-point bending strength was measured. In addition, an elastic modulus and a strain of the test piece were calculated from the results of the three-point bending strength.

Table 1 indicates the results of the aligner crack test and the three-point bend test of the test piece.

**[Table 1]**

| | Examples | | | | | | Comparative Examples | | | |
|---|---|---|---|---|---|---|---|---|---|---|
| | 1 | 2 | 3 | 4 | 5 | 6 | 1 | 2 | 3 | 4 |
| UDMA | 49.5 | 38.1 | 41.3 | 45.0 | 47.1 | 19.7 | 59.3 | 49.5 | 48.3 | 34.2 |
| Bis MEPP 1 | 20.6 | 11.4 | 12.4 | 13.5 | 14.1 | 59.1 | 24.7 | 14.8 | 14.5 | 10.3 |
| Bis MEPP 2 | | 11.4 | 12.4 | 13.5 | 14.1 | | | 14.8 | 14.5 | 10.3 |
| TEGDMA | 12.4 | 15.3 | 16.5 | 18.0 | 18.9 | 19.7 | 14.8 | 19.8 | 19.3 | 13.7 |
| TPO | 0.8 | 0.8 | 0.8 | 0.9 | 0.9 | 1.5 | 1.0 | 1.0 | 1.0 | 0.7 |
| BHT | 0.1 | 0.1 | 0.1 | 0.1 | 0.1 | 0.1 | 0.1 | 0.1 | 0.1 | 0.1 |
| Polyurethane powder (C-400) | 16.5 | 22.9 | 16.5 | 9.0 | 4.7 | | | | 2.4 | 30.8 |
| Polyurethane powder (C-1000) | | | | | | 19.7 | | | | |
| Total | 100 | 100 | 100 | 100 | 100 | 100 | 100 | 100 | 100 | 100 |
| Viscosity of paste [mPa·s] | 1700 | 1810 | 1060 | 640 | 480 | 680 | 670 | 380 | 410 | 3550 |
| Aligner crack test | Excellent | Excellent | Excellent | Excellent | Excellent | Excellent | Poor | Poor | Poor | - |

| Bending test of test piece | | | | | | | | | | |
|---|---|---|---|---|---|---|---|---|---|---|
| Three-point bending strength [MPa] | 47.6 | 47.0 | 60.9 | 70.4 | 77.2 | 19.8 | 80.8 | 88.0 | 81.6 | - |
| Elastic modulus [GPa] | 0.9 | 0.7 | 1.2 | 1.5 | 1.8 | 0.2 | 1.9 | 2.1 | 1.9 | - |
| Strain [%] | 18.8 | 18.5 | 15.1 | 12.6 | 12.8 | 18.0 | 13.5 | 8.0 | 10.1 | - |

From Table 1, it can be seen that when the orthodontic aligner was three-dimensionally produced using the three-dimensional printing paste of Examples 1 to 6, the generation of cracks during attachment and detachment can be suppressed.

In contrast, when the orthodontic aligner was three-dimensionally produced, cracks were generated during attachment and detachment because the three-dimensional printing paste of Comparative Examples 1 and 2 did not contain the polyurethane powder.

When the orthodontic aligner was three-dimensionally produced, cracks were generated during attachment and detachment, because the three-dimensional printing paste of Comparative Example 3 contained 2.4% by mass of polyurethane powder.

An orthodontic aligner was not able to be produced by the three-dimensional printing because the viscosity of the three-dimensional printing paste in Comparative Example 4 was 3,550 mPa·s.

This international application is based on and claims priority of Japanese Patent Application No. 2018-136108 filed July 19, 2018.

## Claims

1. A three-dimensional printing composition comprising:
10 to 80% by mass of (meth)acrylate having a urethane bond;
10 to 80% by mass of (meth)acrylate free from a urethane bond;
3 to 30% by mass of a polyurethane powder; and
0.01 to 10% by mass of a photopolymerization initiator,
wherein the viscosity of the three-dimensional printing composition measured using a type B viscometer at 23°C is 200 to 3,000 mPa·s.

2. Use of the composition according to claim 1 for forming a three-dimensional printing of an orthodontic aligner.

3. A method of producing a dental article, the method comprising:
producing a dental article by three-dimensional printing using the three-dimensional printing composition of claim 1.

## Patentansprüche

1. Zusammensetzung zum dreidimensionalen Drucken, umfassend:
10 bis 80 Massen-% (Meth)acrylat mit einer Urethanbindung;
10 bis 80 Massen-% (Meth)acrylat, das frei von einer Urethanbindung ist;
3 bis 30 Massenprozent eines Polyurethanpulvers; und
0,01 bis 10 Masse-% eines Photopolymerisationsinitiators,
wobei die Viskosität der Zusammensetzung zum dreidimensionalen Drucken, gemessen mit einem Viskosimeter vom Typ B bei 23 °C, 200 bis 3.000 mPa · s beträgt.

2. Verwendung der Zusammensetzung nach Anspruch 1 zur Herstellung eines dreidimensionalen Drucks eines kieferorthopädischen Aligners.

3. Verfahren zur Herstellung eines Dentalartikels, wobei das Verfahren umfasst:
Herstellen eines Dentalartikels durch dreidimensionales Drucken unter Verwendung der Zusammensetzung zum dreidimensionalen Drucken nach Anspruch 1.

## Revendications

1. Composition pour impression tridimensionnelle comprenant :
10 à 80 % en masse de (méth)acrylate ayant une liaison uréthane ;
10 à 80 % en masse de (méth)acrylate exempt de liaison uréthane ;
3 à 30 % en masse d'une poudre de polyuréthane ; et
0,01 à 10 % en masse d'un initiateur de photopolymérisation,
dans laquelle la viscosité de la composition pour impression tridimensionnelle mesurée en utilisant un viscosimètre de type B à 23 °C est de 200 à 3000 mPa·s.

2. Utilisation de la composition selon la revendication 1 pour former une impression tridimensionnelle d'une gouttière orthodontique.

3. Procédé de production d'un article dentaire, le procédé comprenant :
produire un article dentaire par impression tridimensionnelle en utilisant la composition pour impression tridimensionnelle selon la revendication 1.
